Europäisches Patentamt

(19) European Patent Office

Office européen des brevets

(11) Numéro de publication : **0 248 832 B1**

(12) **FASCICULE DE BREVET EUROPEEN**

(45) Date de publication du fascicule du brevet :
29.05.91 Bulletin 91/22

(21) Numéro de dépôt : 86906866.8

(22) Date de dépôt : 04.12.86

(86) Numéro de dépôt international :
PCT/FR86/00417

(87) Numéro de publication internationale :
WO 87/03616 18.06.87 Gazette 87/13

(51) Int. Cl.⁵ : **C12N 1/00, C12N 1/04, C12N 1/20, C12N 1/38, A01G 7/00, A01N 63/00, C05F 11/08**

(54) PROCEDE DE CULTURE DE MICROORGANISMES, NOTAMMENT DU GROUPE DES FRANKIA ET PREPARATION D'INOCULUMS BACTERIENS.

(30) Priorité : 04.12.85 FR 8517933

(43) Date de publication de la demande :
16.12.87 Bulletin 87/51

(45) Mention de la délivrance du brevet :
29.05.91 Bulletin 91/22

(84) Etats contractants désignés :
AT BE CH DE FR GB IT LI LU NL SE

(56) Documents cités :
EP-A- 0 083 267
WO-A-81/01714
WO-A-85/05630
FR-A- 1 466 843
FR-A- 2 086 927
FR-A- 2 501 229
US-A- 4 518 693
Plant and Soil, vol. 87, 1985, Martinus Nijhoff Publishers, (Dordrecht, NL); H.G. Diem et al.:"In vitro production of specialized reproductive torulose hyphase by Frankia strain ORS 021001 isolated from Casuarina junghuhniana root nodules", pp. 17-29

(56) Documents cités :
Journal of Biotechnology, vol. 2, 1985, Elsevier Science Publishers B.V. (NL); H.Nakajima et al.:"Entrapment of Lavandula vera cells and production of pigments by entrapped cells", pp. 107-117
Nature, vol. 302, no. 5909, avril 1983 (Chesham, Bucks, GB) K. Nilsson et al.:"Entrapment of animal cells for production of monoclonal antibodies and other biomolecules", pp. 629-630

(73) Titulaire : INSTITUT FRANCAIS DE RECHERCHE SCIENTIFIQUE POUR LE DEVELOPPEMENT EN COOPERATION (ORSTOM)
24, rue Bayard
F-75008 Paris (FR)

(72) Inventeur : HOANG, Gia, Diem
Orstom B.P. 1386
Dakar (SN)
Inventeur : DOMMERGUES, Yvon, René
76, chaussée de l'Etang
F-94160 St. Mandé (FR)

(74) Mandataire : Warcoin, Jacques et al
Cabinet Régimbeau 26, avenue Kléber
F-75116 Paris (FR)

## Description

La présente invention concerne un procédé de culture de microorganismes ainsi qu'un procédé de fabrication d'un inoculum à partir des microorganismes obtenus.

Plus particulièrement, la présente invention concerne la production d'inoculums nécessaires à l'horticulture et à la foresterie (par exemple l'obtention en pépinière des plants inoculés de Casuarina, aulne, Hippophaë, Elaeagnus).

Parmi les microorganismes utilisables à titre d'inoculum, il faut citer les actinomycètes appartenant au groupe des Frankia (symbiotes des plantes actinorhiziennes fixatrices d'azote) et les rhizobiums.

Certains microorganismes, notamment les Frankia, présentent la particularité de produire une très faible biomasse lorsqu'on les cultive suivant les méthodes usuelles. Cette très faible production de biomasse constitue un grave handicap, à la fois dans le domaine industriel et dans le domaine de la recherche fondamentale.

C'est pourquoi la présente invention propose un procédé de culture de microorganisme et de fabrication d'un inoculum permettant la production de biomasses beaucoup plus importante que celles qui sont obtenues actuellement, ainsi que les processus permettant de satisfaire aux besoins industriels des horticulteurs par exemple.

En effet, compte tenu de leur utilisation, les inoculums bactériens destinés à l'horticulture ou à la foresterie doivent remplir certaines conditions :

– Ils doivent tout d'abord être constitués d'un support défini et assurant une bonne protection des microorganismes considérés ;

– ils doivent être faciles à stocker, à expédier et à utiliser ;

– enfin, permettre, lors de leur application, une très large dissémination des microorganismes au niveau des sites d'infection des plantes hôtes.

Plus particulièrement, la présente invention concerne des inoculums constitués de microorganismes inclus dans des matrices polymères. De tels procédés,ont déjà été décrits, notamment dans les demandes de brevet français suivantes : 77 10254-79 28956-81 04474-83 02847.

Toutefois, aucun de ces procédés ne permet de remplir de façon totalement satisfaisante les conditions évoquées précédemment.

Pour ce faire, la présente invention propose un procédé de culture de microorganismes, caractérisé en ce qu'il s'agit d'une culture biphasique dans laquelle :

a) on cultive les microorganismes sur un milieu nutritif solide (étape A dudit procédé), et après culture on fragmente ce milieu solide colonisé par les microorganismes,

b) on ensemence avec les fragments obtenus à l'étape précédente un milieu nutritif liquide (étape B dudit procédé) et après croissance des microorganismes à l'interface solide-liquide dudit milieu nutritif liquide on récupère ceux-ci.

Ce procédé est en particulier utilisable pour la préparation d'Actinomycètes à croissance lente, notamment ceux appartenant au groupe des Frankia.

Le procédé selon la présente invention met à profit le fait que certains microorganismes, en particulier les Frankia, croissent beaucoup mieux au niveau d'une interface solide/liquide qu'au sein d'un milieu liquide.

Ainsi, par exemple, une culture biphasique liguide/solide avec des fragments de gélose permet, non seulement l'adhésion de Frankia à leur surface en raison du caractère thigmotrope de ce microorganisme (caractère observé chez certains autres microorganismes ainsi que cela a déjà été signalé), mais également la pénétration de Frankia à l'intérieur de ces supports. Le milieu liquide participe, non seulement à la réalisation de l'interface solide/liquide favorable au développement microbien, mais constitue aussi la réserve de nutriments.

Les milieux nutritifs solides ou liquides peuvent avoir sensiblement les mêmes compositions et constituent, pour l'essentiel, des milieux connus ou des milieux dérivés de milieux connus contenant du carbone, de l'azote, du phosphore, des oligoéléments et autres éléments assurant la croissance du microorganisme qu'on entend cultiver.

L'obtention d'un milieu solide peut être réalisée en utilisant de la gélose, mais d'autres composants peuvent être utilisés pour obtenir un milieu solide.

"L'étape a" peut être conduite dans un récipient du type boîte de Petri. Après culture le milieu solide est fragmenté par des moyens appropriés puis dispersé dans un milieu nutritif liquide. En fin de croissance, les microorganismes obtenus sont récupérés par des techniques connues telles que la centrifugation.

On a constaté, en outre, que l'adjonction au milieu nutritif de charbon activé, en particulier lorsque l'adjonction est effectuée au niveau du milieu solide ("étape a"), permet d'augmenter notablement la biomasse.

Afin d'améliorer encore la qualité de l'inoculum final, on opère de préférence de la façon suivante :

a) on inclut les microorganismes dans une matrice de polymère ("étape a" dudit procédé) ;

2

b) ces matrices incluant les microorganismes obtenus à l'issue de "l'étape a" précédente sont incubées dans un milieu permettant la croissance desdits microorganismes jusqu'à la formation de colonies à l'intérieur desdites matrices ("étape b" dudit procédé) ;

c) les matrices de polymère incluant les colonies de microorganismes obtenues au cours de "l'étape b" précédente sont déshydratées ("étape c" dudit procédé).

Les matrices de polymère qui peuvent être mises en oeuvre sont connues de l'homme de métier et figurent notamment dans les brevets mentionnés précédemment. Il pourra s'agir, en particulier, de billes d'alginate de calcium qui sont particulièrement bien appropriées à l'obtention d'un inoculum microbien.

On sait que les microorganismes inclus incubés dans un milieu permettant la croissance sont capables de poursuivre leur croissance à l'intérieur de leur matrice de polymère. On a observé maintenant qu'ils étaient capables, dans cette matrice de polymère, de former des microcolonies, ce qui présente de nombreux avantages. Tout d'abord, on obtient un inoculum à forte densité, mais également la structure fine en microcolonie présente comme avantage de protéger l'inoculum lors de son stockage.

De préférence, pour le stockage, ces matrices de polymère sont déshydratées sans être fractionnées. Ainsi, lorsque l'on prépare des billes d'alginate de calcium, celles-ci ne sont pas fragmentées mais déshydratées telles quelles.

Dans un mode de réalisation préféré, les microorganismes inclus dans la matrice de polymère sont obtenus par le procédé décrit précédemment. Les fragments, par exemple de gélose, contenant les microorganismes tels que Frankia, sont incorporés dans la matrice de polymère, par exemple l'alginate de calcium.

Enfin, lorsque l'inoculum microbien préalablement déshydraté doit être appliqué in situ, on le réhydrate avec un tampon, par exemple un tampon phosphate, jusqu'à obtention d'un gel. Ce gel se prête particulièrement bien à la dissémination des microorganismes dans le sol.

Le procédé décrit précédemment permet d'obtenir un inoculum qui, après déshydratation, peut être stocké et transporté sans difficulté, qui présente une biomasse importante et qui peut être dispersé très aisément sous forme du gel.

Les exemples ci-dessous permettront de mettre en évidence d'autres avantages et caractéristiques de la présente invention.

Les milieux de culture utilisés dans ces exemples ont les compositions suivantes :

## 1. Milieu Qmod  (Lalonde et Calvert, 1979)

| Composants | Concentration |
|---|---|
| $K_2HPO_4$ | 0,3 g |
| $NaH_2PO_4$ | 0,2 g |
| $MgSO_4$ $7H_2O$ | 0,2 g |
| KCl | 0,2 g |
| Extrait de levure | 0,5 g |
| Peptone | 5,0 g |
| Citrate ferrique (solution à 1 %) | 1 ml |
| Solution d'oligoéléments * | 1 ml |
| Lécithine | 5 mg |
| Eau distillée | 1 l |

pH ajusté à 6,8

* Solution d'oligoéléments (g/l)

$H_3BO_3$ : 1,5 ; $MnSO_4 7H_2O$ : 0,8 ; $ZnSO_4 7H_2O$ : 0,6 ;
$CuSO_4 7H_2O$ : 0,1 ; $(NH_4)_6Mo_7O_{24} 4H_2O$ : 0,2 ;
$CoSO_4 7H_2O$ : 0,01.

3

2. <u>Milieu Qmod + charbon activé</u> (Diem et Dommergues, 1985)

Milieu Qmod précédent

additionné de charbon activé
à la dose de 150 mg/l

3. <u>Milieu BAP</u> (Murry et al., 1984)

| <u>Composants</u> | <u>Concentration</u> | |
|---|---|---|
| $K_2HPO_4$ | 0,591 | g |
| $KH_2PO_4$ | 0,952 | g |
| $NH_4Cl$ | 0,267 | g |
| $MgSO_4\ 7H_2O$ | 0,095 | g |
| $CaCl_2\ 2H_2O$ | 0,010 | g |
| FeNa EDTA | 0,010 | g |
| Pyruvate de sodium | 1,1 | g |
| Solution d'oligoéléments * | 1 | ml |
| Solution de vitamines ** | 1 | ml |
| Eau distillée | 1 | l |

pH ajusté à 6,3

\* Solution d'oligoéléments (g/l)

$H_3Bo_3$ : 2,86 ; $MnCl_24H_2O$ : 2,27 ; $ZnSO_47H_2O$ : 0,22 ;
$CuSO_45H_2O$ : 0,08 ; $Na_2MoO_42H_2O$ : 0,025 ;
$CoSO_47H_2O$ : 0,001.

\*\* Solution de vitamines (mg/l)

Thiamine HCl : 10 ; acide nicotinique : 50 ;

pyridoxine HCl : 50 ; biotine : 225 ;

acide folique : 10 ; pantothénate de calcium : 10 ;

riboflavine : 10.

4. <u>Milieu YEM</u> (Yeast extract-mannitol) :

| <u>Composants</u> | <u>Concentration</u> | |
|---|---|---|
| $K_2HPO_4$ | 0,5 | g |
| $MgSO_4\ 7H_2O$ | 0,2 | g |
| NaCl | 0,1 | g |
| Yeast extract (Difco) | 1,0 | g |
| Mannitol | 10,0 | g |
| Eau distillée | 1 | l |

5. <u>Milieu YEM modifié</u>

Même composition que précédemment sauf $K_2HPO_4$ qui est utilisé à la concentration de 0,1 g/l.
Sur les figures,
– la figure 1 représente l'effet de l'adjonction de charbon actif au milieu de culture sur la croissance de <u>Frankia</u> :

| 1a : | milieu Qmod seul ; |
| 1b : | même milieu avec charbon actif (0,01%) ; les points noirs (flèches) apparaissant sur les boîtes de Petri sont les colonies de <u>Frankia</u> (la poudre de charbon n'est pas visible à cette échelle). |

– la figure 2 représente des billes d'alginate colonisées à l'intérieur par des <u>Frankia</u> :

| 2a : | vue de trois billes (diamètre réel : environ 5 mm) ; |
| 2b : | vue agrandie d'une bille où les colonies de <u>Frankia</u> (diamètre réel de chaque colonie : 100-200 µm) apparaissent comme des taches circulaires noires à bord diffus ; autour de certaines colonies on observe des amas de points noirs qui sont des sporanges formés pendant la croissance de <u>Frankia</u> à l'intérieur de la bille ; les sporanges (flèches) sont des structures typiques de <u>Frankia</u> ; |

## <u>EXEMPLE 1 – PROCEDE DE CULTURE EN DEUX ETAPES ET EN MILIEU BIPHASIQUE DE CERTAINS ACTINOMYCETES A CROISSANCE LENTE APPARTENANT AU GROUPE DES FRANKIA</u>

La culture se fait en deux étapes :
"Etape a" – On cultive <u>Frankia</u> en boîte de Petri (10 cm) dans un milieu gélosé pendant 2 à 3 semaines. Pour ce faire, on ensemence, dans la masse, 10 ml de milieu nutritif gélosé à 1,5% fondu à 40°C avec 1 ml d'une suspension de <u>Frankia</u> obtenue par homogénéisation d'une culture de <u>Frankia</u>. Après 2-3 semaines d'incubation à 30°C, les colonies de <u>Frankia</u> se sont développées dans la gélose (500 à 2000 colonies par boite). Chacune de ces plaques de gélose envahies de colonies de <u>Frankia</u> est détachée de sa botte de Petri et introduite stérilement dans une fiole contenant 50 ml d'eau et un barreau magnétique.
Les plaques sont ainsi fragmentées finement en microgranules de gélose qui renferment des colonies ou fragments de colonies de <u>Frankia</u>.
"Etape b" – La suspension de fragments obtenue comme indiqué ci-dessus est utilisée pour ensemencer un milieu nutritif liquide à raison de 10 ml de suspension de fragments <u>Frankia</u> par 50 ml de milieu nutritif liquide. L'incubation à 30°C dure 2 à 3 semaines. La composition du milieu nutritif (gélosé ou liquide) varie suivant les souches de <u>Frankia</u> utilisées. Il peut s'agir soit du milieu Qmod, soit du milieu BAP.

<u>Détermination de la biomasse de Frankia obtenue</u>

Etant donné que Frankia est un organisme filamenteux, il n'est pas possible d'estimer la biomasse en nombre de cellules comme c'est le cas pour les bactéries. On l'exprime soit en poids de protéines (ce qui est la solution adoptée ici), soit en poids de la matière sèche.
Après élimination du milieu nutritif par centrifugation, on introduit le culot de centrifugation dans un tube à essai contenant environ 30 ml d'eau distillée et on met au bain marie en ébullition pendant 2 min pour faire fondre la gélose des microgranules. On verse ensuite tout le contenu du tube dans environ 200 ml d'eau distillée bouillante pour diluer la gélose fondue afin de pouvoir l'éliminer, soit par filtration sur filtre Millipore, soit par centrifugation. On rince la culture plusieurs fois à l'eau distillée puis on la récolte pour effectuer le dosage de protéines selon la méthode de Lowry et al. (1951).

<u>Comparaison de la méthode usuelle en milieu liquide et de la méthode selon l'invention en milieu biphasique</u>

Afin de comparer ces deux méthodes, on a été amené à conduire la culture en milieu liquide en adoptant la même succession de manipulations que celle préconisée dans le cadre de la présente invention.
Le tableau 1 résume la succession des opérations dans les deux cas.

## TABLEAU 1

### COMPARAISON DE LA METHODE DE CULTURE USUELLE EN MILIEU LIQUIDE

### ET DE LA METHODE DE CULTURE BIPHASIQUE SELON L'INVENTION AVEC DES FRAGMENTS DE GELOSE

| Méthode de culture usuelle en milieu liquide | Méthode de culture biphasique avec des fragments de gélose |
|---|---|
| 1 ml de la suspension de cellules de **Frankia** | 1 ml de la suspension de cellules de **Frankia** |
| ↓ | ↓ |
| Culture sur milieu liquide pendant 2 à 3 semaines | Culture sur milieu gélosé pendant 2 à 3 semaines |
| ↓ | ↓ |
| Homogénéisation de la culture | Homogénéisation de la culture et obtention de fragments de gélose renfermant les colonies de **Frankia** |
| ↓ | ↓ |
| Continuation de la culture en milieu liquide pendant 2 à 3 semaines | Transfert des fragments en milieu liquide et culture pendant 2 à 3 semaines (culture biphasique) |
| ↓ | ↓ |
| Centrifugation, lavage | Centrifugation, lavage |
| ↓ | ↓ |
| Produit final | Produit final |

EP 0 248 832 B1

Culture de la souche de Frankia de Casuarina equisetifolia ORS 021001

Les résultats qui figurent au tableau 2 montrent clairement que le procédé selon l'invention permet l'obtention d'une biomasse beaucoup plus importante (× 20) tout en partant d'un inoculum identique et ce pour la même durée d'incubation.

Culture de trois autres souches de Frankia

Pour vérifier la fiabilité du procédé selon l'invention, on a refait cette expérience à un autre moment de l'année avec trois autres souches de Frankia qui sont les suivantes :

ORS 022602 :    Frankia d'Allocasuarina stricta
ORS 060501 :    Frankia de Colletia spinosa
ORS 140102 :    Frankia d'Hippophaë rhamnoides.

Les résultats qui figurent au tableau 3 confirment l'amélioration de la production de la biomasse de Frankia par le procédé de culture selon l'invention par rapport à la méthode de culture usuelle. Cette amélioration est plus faible dans cet essai que dans l'essai précédent.
Ceci serait dû à plusieurs causes :
● temps d'incubation réduit à 4 semaines au lieu de 6 semaines,
● état physiologique et présence variable de diverses structures de Frankia (hyphes végétatifs, sporanges) dans l'inoculum initial.

## TABLEAU 2

### COMPARAISON DE LA CROISSANCE DE FRANKIA ORS 021001 CULTIVEE SELON LA METHODE USUELLE EN MILIEU LIQUIDE ET SELON LA NOUVELLE METHODE DE CULTURE BIPHASIQUE AVEC MICROGRANULES DE GELOSE

| METHODE | BIOMASSE DE FRANKIA (µg protéines/fiole) | |
| --- | --- | --- |
| | A L'ENSEMENCEMENT | APRES 6 SEMAINES D'INCUBATION (1) |
| Méthode usuelle | 2 | 55 |
| Méthode de culture biphasique | 2 | 1 205 |

(1) dont 3 semaines sur milieu gélosé et 3 semaines en milieu biphasique

Milieu nutritif utilisé : Qmod

## TABLEAU 3

### COMPARAISON DE LA CROISSANCE DE TROIS SOUCHES DE FRANKIA CULTIVEES SELON LA METHODE USUELLE EN MILIEU LIQUIDE ET SELON LA NOUVELLE METHODE DE CULTURE BIPHASIQUE AVEC FRAGMENTS DE GELOSE

| METHODE ET SOUCHE | BIOMASSE DE FRANKIA (µg protéines/fiole) | |
| --- | --- | --- |
| | A L'ENSEMENCEMENT | APRES 4 SEMAINES D'INCUBATION (1) |
| Souche ORS 022602 | | |
| . Méthode usuelle | 2 | 26 |
| . Méthode biphasique | 2 | 145 |
| Souche ORS 060501 | | |
| . Méthode usuelle | 3 | 58 |
| . Méthode biphasique | 3 | 315 |
| Souche ORS 140102 | | |
| . Méthode usuelle | 6 | 41 |
| . Méthode biphasique | 6 | 360 |

(1) dont 2 semaines sur milieu gélosé et
    2 semaines en milieu biphasique

Milieux nutritifs utilisés : même milieu que dans le
tableau 2, sauf pour ORS 022602 qui a été cultivé sur
milieu BAP

## EXEMPLE 2 – EFFET DE L'ADJONCTION DE CHARBON ACTIVE AU MILIEU DE CULTURE Qmod SUR LE NOMBRE DE COLONIES DE FRANKIA SOUCHE ORS 021001

On cultive la souche ORS 021001 dans des tubes à essai de 18 × 180 mm contenant 10 ml de milieu Qmod. L'incubation a lieu à 30°C et les durées d'incubation sont respectivement de 3 semaines, 1 mois et 2 mois. A la fin de ces périodes d'incubation, chacun des tubes renferme de 50 à 70 µg de protéines de Frankia.

On décante chaque culture, on ajoute 20 ml de milieu Qmod frais et on homogénéise stérilement à l'aide d'un barreau magnétique pendant 1 heure. On fait deux dilutions ($10^{-1}$ et $10^{-2}$) des suspensions obtenues et on inocule deux séries de boîtes de Petri en incorporant 0,5 ml de chaque suspension-dilution à 20 ml de milieu Qmod gélosé et additionné ou non de charbon activé (Merck Art. 2186) à la dose de 150 mg par litre. On incube les boîtes de Petri ainsi ensemencées à 30°C pendant 3 semaines. On compte le nombre de colonies formées sous une loupe binoculaire, les résultats étant exprimés sur la base de 1 ml d'inoculum.

Le tableau 4 montre clairement que l'adjonction de charbon au milieu accroît le nombre de colonies de Frankia observées, cet accroissement étant considérable dans le cas des cultures de 2 mois.

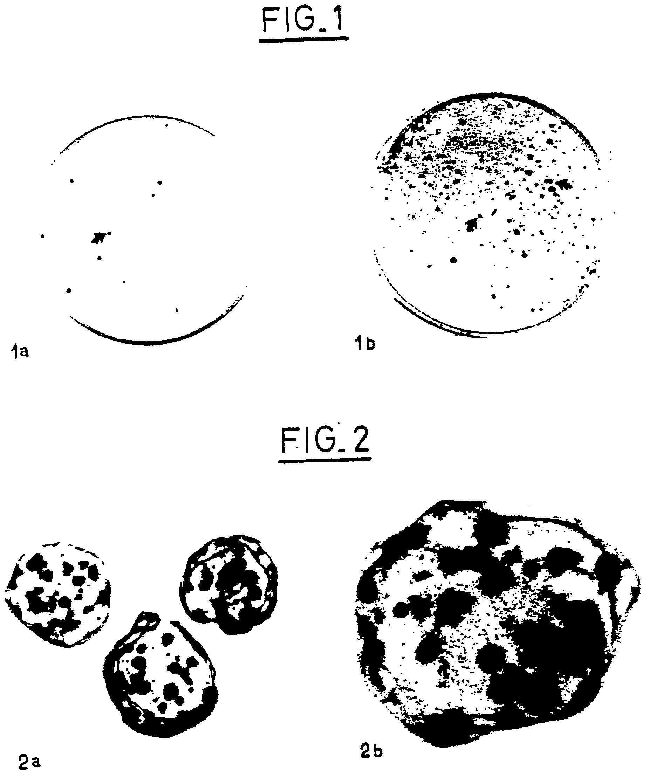

Ce premier résultat indique que le charbon activé favorise la germination des structures de type spore accumulées dans les cultures de 2 mois (figure 1). D'un autre côté, l'observation à la loupe montre que les hyphes nouvellement formés sont plus ramifiés sur milieu Qmod avec charbon activé que sur milieu Qmod seul, ce deuxième résultat prouvant que le charbon activé favorise la croissance des hyphes.


## TABLEAU 4


## EFFET DE L'ADJONCTION DE CHARBON ACTIVE AU MILIEU NUTRITIF (Qmod) SUR LE NOMBRE DE COLONIES FORMEES A PARTIR DE 1 ml DE SUSPENSION DE FRANKIA ORS 021001


|  | NOMBRE DE COLONIES FORMEES | |
| --- | --- | --- |
| CULTURE DE FRANKIA | MILIEU Qmod | MILIEU Qmod + charbon |
| Culture de 3 semaines | 1 000 | 3 400 |
| Culture de 1 mois | 8 200 | 266 000 |
| Culture de 2 mois | 4 200 | 940 000 |


## EXEMPLE 3 – CROISSANCE DE FRANKIA DANS LES BILLES D'ALGINATE

On ensemence 100 ml de milieu Qmod avec 10 ml d'une suspension de cellules de Frankia ORS 021001. Après 4 semaines d'incubation, on obtient une culture renfermant une biomasse de Frankia qui, évaluée en protéines (Lowry et al., 1951), atteint 280 µg. On décante la culture, on la transfère dans la même quantité (100 ml) de milieu Qmod liquide frais, on homogénéise avec un barreau magnétique pendant 1 heure. On procède alors à l'inclusion de Frankia dans l'alginate. Pour ce faire, on ajoute 100 ml de milieu Qmod liquide stérile contenant l'alginate à 4% S170 (Satialgine S170, Société Bretonne de Produits Chimiques et Pharmaceutiques, 6 impasse Latécoère, Velizy 78140). On obtient ainsi au total 200 ml de suspension de fragments de colonies de Frankia dans le milieu Qmod contenant 2% d'alginate. On laisse tomber goutte à goutte aseptiquement cette suspension dans un récipient contenant une solution aqueuse stérile de $CaCl_2$ (1%) et muni d'un barreau magnétique en agitation continue.

Dans ces conditions, les billes se forment au bout de 20 à 30 min dans la solution de $CaCl_2$. Les billes sont immédiatement rincées 10 fois avec de l'eau distillée stérile.

On transfère les billes dans les fioles contenant 50 ml de milieu Qmod liquide (environ 20 ml de billes par fiole) et on incube à 30°C pendant 8 semaines. Après ce délai, l'observation à la loupe montre que de nombreuses colonies de Frankia (20-40 colonies/bille) se sont développées à l'intérieur de chaque bille parfaitement dépourvue de toute contamination microbienne exogène. Ces colonies ont des tailles différentes mais sont parfaitement identiques en ce qui concerne la morphologie et le mode de croissance radiale typique de Frankia. Beaucoup de colonies produisent des sporanges également typiques (figure 2).

## EXEMPLE 4 – CROISSANCE DE RHIZOBIUM DANS LES BILLES D'ALGINATE

On fait une culture de Rhizobium souche A16 isolée d'Albizia lebbeck sur un milieu YEM usuel. Après 4 jours d'incubation, on prélève 1 ml que l'on introduit dans 100 ml de milieu YEM modifié, ce milieu est pauvre en phosphate et contient de l'alginate S170 (Satialgine S170, Société Bretonne de Produits Chimiques et Pharmaceutiques, 6 impasse Latécoère, Velizy 78140). On obtient des billes en effectuant les opérations de l'exemple 3. On fait deux lots de ces billes contenant les cellules de Rhizobium inclus :
- le premier lot est immergé dans une fiole renfermant du milieu nutritif liquide YEM modifié mais dépourvu d'alginate ;
- le deuxième lot est incubé dans les mêmes conditions mais en l'absence de milieu nutritif.

Au bout de 48 heures, on constate que le nombre de Rhizobium dénombré sur milieu Yem normal gélosé passe de $175.10^3$ par bille à $280.10^5$ par bille dans le premier lot, alors que dans le deuxième lot le nombre de Rhizobium reste stationnaire. Ce résultat montre que la croissance de Rhizobium à l'intérieur des billes d'alginate requiert, comme dans le cas de Frankia, l'incubation de ces billes dans un milieu nutritif liquide approprié.

## EXEMPLE 5 – REHYDRATATION DES BILLES D'ALGINATE DESHYDRATEES

Les billes d'alginate renfermant les microorganismes symbiotiques préalablement déshydratées sont immergées dans une solution de tampon phosphate (pH 6,8) ayant la composition suivante :
$KH_2PO_4$ : 4,29 g ; $K_2HPO_4$ : 4,34 g ; eau q.s.p. 1 litre.

Au bout de 4 à 6 heures, les billes déshydratées reprennent la forme et la consistance initiales des billes fraîches. Par écrasement, il est très facile d'obtenir l'inoculum sous forme d'un gel. Ce traitement au tampon phosphate est indispensable car il permet la libération et la dissémination des microorganismes précédemment inclus ou développés après l'inclusion.

## EXEMPLE 6 – INOCULATION DES PLANTES

Pour contrôler la qualité de l'inoculum obtenu par le procédé selon l'invention, on a testé l'infectivité d'un inoculum de Frankia sur Casuarina equisetifolia (Frankia souche ORS 021001) et d'un inoculum de champignon endomycorhizien vésiculo-arbusculaire (Glomus mosseae).

### Frankia souche ORS 021001

On a d'abord obtenu des plantules de Casuarina equisetifolia en faisant germer des graines superficiellement stérilisées (trempage dans $H_2SO_4$ concentré pendant 1 min, puis rinçage abondant avec de l'eau distillée stérile). Les plantules âgées de 4 semaines ont été repiquées à raison de 1 plantule par pot en plastique (diamètre 7 cm, hauteur 7 cm) contenant du sol stérile humidifié quotidiennement à l'eau stérile. Pour inoculer les plantules au moment du repiquage, on prépare l'inoculum comme suit :
- on fait regonfler 120 mg de billes déshydratées dans le tampon phosphate indiqué ci-dessus ;
- après écrasement on mélange le gel obtenu avec du sable stérile et l'on répartit en 12 pots, ce qui revient à apporter à chaque pot inoculé 10 mg de billes déshydratées.

On a utilisé deux lots d'inoculum : un premier lot stocké pendant 2 semaines seulement (inoculum n° 1), un deuxième lot stocké à la température du laboratoire pendant 1 an (inoculum n° 2). Bien entendu, parallèlement aux pots inoculés, on a mis en place une série de pots témoins non inoculés. Après 2 mois de culture, on détermine le poids sec des parties aériennes des plantes (séchage jusqu'à poids constant), le nombre de plantes nodulées et le nombre de nodules par plante (tableau 5).

## TABLEAU 5

### INOCULATION DES PLANTULES DE CASUARINA EQUISETIFOLIA AVEC ORS 021001 INCLUS DANS LES BILLES D'ALGINATE DESHYDRATEES PUIS REGONFLEES AU MOMENT DE L'EMPLOI DANS LE TAMPON PHOSPHATE

| TRAITEMENT | POIDS SEC DES PLANTES (mg/plante) | PLANTES NODULEES SUR NOMBRE TOTAL | NOMBRE DES NODULES/PLANTE |
|---|---|---|---|
| Témoin | 80 | 0 / 12 | 0 |
| Inoculum n°1 | 220 | 12 / 12 | 3 - 8 |
| Inoculum n°2 | 170 | 12 / 12 | 3 - 5 |

Inoculum n° 1    Billes conservées sous forme déshydratée pendant 2 semaines à la température du laboratoire

Inoculum n° 2    Billes conservées sous forme déshydratée pendant 1 an à la température du laboratoire.

### Glomua mosseae

On a inclus dans les billes d'alginate les structures de Glomus mosseae (hyphes libres ou dans les racines, spores) suivant la méthode décrite par Diem et al. (1981) et Ganry et al. (1982). On a repiqué des plantules de Vigna unguiculata âgées de 7 jours dans des pots en terre cuite (diamètre 15 cm, hauteur 20 cm) contenant du sol stérile. Un premier lot de 5 plantes a été inoculé avec un inoculum qui est un mélange de sable et de gelée préparéé de la même façon que précédemment décrit mais à partir de billes déshydratées contenant les structures de Glomus mosseae. Ces billes avaient été stockées pendant 1 an à la température du laboratoire. On a apporté à chaque pot inoculé l'équivalent de 30 mg de billes déshydratées. Un deuxième lot de 5 plantes a été constitué par les plantes non inoculées.

Le tableau 6 montre que l'inoculum de Glomus mosseae est parfaitement infectif à la dose utilisée et après conservation pendant 1 an.

11

## TABLEAU 6

### INOCULATION DES PLANTULES DE VIGNA UNGUICULATA AVEC GLOMUS MOSSEAE INCLUS DANS LES BILLES D'ALGINATE DESHYDRATEES PUIS REGONFLEES AU MOMENT DE L'EMPLOI DANS LE TAMPON PHOSPHATE

| TRAITEMENT | PLANTES INFECTEES SUR NOMBRE TOTAL | FREQUENCE D'INFECTION (%) |
|---|---|---|
| Témoin | 0 / 5 | 0 |
| Inoculum n° 1 | 5 / 5 | 86 |

Inoculum n° 1 : Billes conservées sous forme déshydratée pendant 1 an

Fréquence d'infection : nombre de morceaux de racine (3 mm) infectés sur nombre total observé

REFERENCES

DIEM H.G. et Y.R. DOMMRGUES (1985) In vitro production of specialized reproductive torulose hyphae by Frankia strain ORS 021001 isolated from Casuarina junghuhniana root nodules. Plant and Soil 87 : 17-29.

LALONDE M. et H.E. CALVERT (1979) Production of Frankia hyphae and spores as an infective inoculant for Alnus species. In : Symbiotic Nitrogen Fixation in the Management of Temperate Forests. (J.C. Gordon, C.T. Wheeler et D.A. Perry, eds) pp. 95-110. (Corvallis, Forest Research Laboratory).

LOWRY O.H., N.J. ROSEBROUGH, A.L. FARR et R.J. RANDALL (1951) Protein measurements with the folin phenol reagent. J. Biol. Chem., 193 : 265-275.

MURRY M.A., M.S. FONTAINE et J.G. TORREY (1984) Growth kinetics and nitrogenase induction in Frankia sp. HFPrl3 grown in batch culture. Plant and Soil, 78 : 61-78.

## Revendications

1. Procédé de culture de microorganismes, caractérisé en ce qu'il s'agit d'une culture biphasique dans laquelle :

a) on cultive les microorganismes sur un milieu nutritif solide (étape A dudit procédé), et après culture on fragmente ce milieu solide colonisé par les microorganismes,

b) on ensemence avec les fragments obtenus à l'étape précédente un milieu nutritif liquide (étape B dudit procédé) et après croissance des microorganismes à l'interface solide-liquide dudit milieu nutritif liquide, on récupère ceux-ci.

2. Procédé selon la revendication 1, caractérisé en ce que le microorganisme cultivé est un Actinomycète à croissance lente, en particulier appartenant au groupe des Frankia.

3. Procédé selon la revendication 1, caractérisé en ce que le milieu nutritif solide utilisé dans l'étape a contient de la gélose.

4. Procédé selon la revendication 1, caractérisé en ce que l'on ajoute au milieu nutritif, de préférence au milieu nutritif solide, du charbon activé.

5. Procédé de préparation d'un inoculum microbien caractérisé en ce qu'il comprend les étapes successives suivantes :

a) on inclut les microorganismes dans une matrice de polymère, ("étape a" dudit procédé) ;

b) les matrices de polymère incluant les microorganismes obtenues à l'issue de "l'étape a" précédente sont incubées dans un milieu permettant la croissance desdits microorganismes jusqu'à la formation de colonies à l'intérieur desdites matrices, ("étape b" dudit procédé) ;

c) les matrices de polymère incluant les colonies de microorganismes obtenues au cours de "l'étape b" précédente sont déshydratées.

6. Procédé de préparation d'un inoculum microbien selon la revendication 5, caractérisé en ce que le polymère utilisé est de l'alginate de calcium.

7. Procédé de préparation d'un inoculum microbien selon l'une des revendications 5 ou 6, caractérisé en ce que les microorganismes sont des microorganismes symbiotiques des plantes.

8. Procédé de préparation d'un inoculum microbien selon la revendication 7, caractérisé en ce que les microorganismes appartiennent au groupe des Frankia ou des Rhizobium.

9. Procédé de préparation d'un inoculum microbien selon l'une des revendications 6 à 8, caractérisé en ce que les matrices de polymère obtenues se présentent sous la forme de billes ou de granules.

10. Procédé de préparation d'un inoculum microbien selon l'une des revendications 5 à 9, caractérisé en ce que les microorganismes à partir desquels on fabrique ledit inoculum sont obtenus par le procédé de culture biphasique selon l'une des revendications 1 à 4.

11. Inoculum microbien caractérisé en ce qu'il est obtenu par le procédé selon l'une des revendications 5 à 10.

12. Application de l'inoculum microbien selon la revendication 11, caractérisée en ce qu'avant utilisation les matrices de polymère sont réhydratées avec un tampon jusqu'à l'obtention d'un gel.


## Ansprüche

1. Verfahren zur Kultur von Mikroorganismen, dadurch gekennzeichnet, daß es sich um eine zweiphasige Kultur handelt, in der man :

a) die Mikroorganismen auf einem festen Nährmedium kultiviert (Stufe A des Verfahrens) und nach der Kultur das feste, durch die Mikroorganismen besiedelte Medium, zerkleinert,

b) mit den in der vorstehenden Stufe erhaltenen Bruchstücken ein flüssiges Nährmedium besät (Stufe B des Verfahrens) und die Mikroorganismen nach dem Wachstum an der Fest-Flüssig-Grenzfläche des flüssigen Nährmediums, gewinnt.

2. Verfahren nach Anspruch 1, dadurch gekennzeichnet, daß der kultivierte Mikroorganismus ein Actinomycet mit langsamem Wachstum, insbesondere zugehörend zur Gruppe der Frankia ist.

3. Verfahren nach Anspruch 1, dadurch gekennzeichnet, daß das in der Stufe a) verwendete feste Nährmedim Gelose enthält.

4. Verfahren nach Anspruch 1, dadurch gekennzeichnet, daß man zu dem Nährmedium, vorzugsweise zum festen Nährmedium, Aktivkohle fügt.

5. Verfahren zur Herstellung eines mikrobiellen Inoculums, dadurch gekennzeichnet, daß es die nachstehenden aufeinanderfolgenden Stufen umfaßt :

a) Man schließt die Mikroorganismen in eine Polymer-Matrix ein ("Stufe a)" des Verfahrens) ;

b) die in der vorstehenden "Stufe a)" erhaltenen, die Mikroorganismen einschließenden Polymer-Matrizes werden in einem Medium inkubiert, das das Wachstum der Mikroorganismen bis zur Bildung von Kolonien im Inneren der Matrizes ermöglicht ("Stufe b)" des Verfahrens) ;

c) die in der vorstehenden "Stufe b)" erhaltenen, die Mikroorganismen-Kolonien einschließenden Polymer-Matrizes werden entwässert.

6. Verfahren zur Herstellung eines mikrobiellen Inoculums nach Anspruch 5, dadurch gekennzeichnet, daß das verwendete Polymere Calciumalginat ist.

7. Verfahren zur Herstellung eines mikrobiellen Inoculums nach einem der Ansprüche 5 oder 6, dadurch gekennzeichnet, daß die Mikroorganismen mit Pflanzen symbiotische Mikroorganismen sind.

8. Verfahren zur Herstellung eines mikrobiellen Inoculums nach Anspruch 7, dadurch gekennzeichnet, daß die Mikroorganismen der Gruppe Frankia oder Rhizobium angehören.

9. Verfahren zur Herstellung eines mikrobiellen Inoculums nach einem der Ansprüche 6 bis 8, dadurch gekennzeichnet, daß die erhaltenen Polymer-Matrizes in der Form von Kugeln oder Granulaten vorliegen.

10. Verfahren zur Herstellung eines mikrobiellen Inoculums nach einem der Ansprüche 5 bis 9, dadurch gekennzeichnet, daß die Mikroorganismen, von denen ausgehend man das Inoculum herstellt, erhalten werden durch das zweiphasige Kulturverfahren nach einem der Ansprüche 1 bis 4.

11. Mikrobielles Inoculum, dadurch gekennzeichnet, daß es erhalten wurde nach dem Verfahren eines der Ansprüche 5 bis 10.

12. Verwendung des mikrobiellen Inoculums gemäß Anspruch 11, dadurch gekennzeichnet, daß vor der Verwendung die Polymer-Matrizes mit einem Puffer bis zur Erzielung eines Gels rehydratisiert werden.

## Claims

1. A process for culturing microorganisms, characterised in that it is a two-phase process wherein :
a) the microorganisms are cultivated on a solid nutrient medium (step A of said process) and after culturing said solid medium colonised by the microorganisms is fragmented,
b) a liquid nutrient medium is seeded (step B of said process) with the fragments obtained in the preceding step, and after the growth of the microorganisms at the solid-liquid interface of said liquid nutrient medium said microorganisms are recovered.

2. A process according to claim 1, characterised in that the microorganism cultivated is a slow-growth Actinomycete, more particularly belonging to the group of the Frankia.

3. A process according to claim 1, characterised in that the solid nutrient medium used in step a contains gelose.

4. A process according to claim 1, characterised in that activated carbon is added to the nutrient medium, preferably to the solid nutrient medium.

5. A process for the preparation of a microbial inoculum, characterised in that it comprises the following successive steps :
a) the microorganisms are enclosed in a polymer matrix ("step a" of said process) ;
b) the polymer matrices enclosing the microorgansims obtained at the end of the preceding "step a" are incubated in a medium allowing the growth of said microorganisms until colonies have formed inside said matrices ("step b" of said process) ;
c) the polymer matrices enclosing the microorganisms colonies obtained during the preceding "step b" are dehydrated.

6. A process for the preparation of a microbial inoculum according to claim 5, characterised in that the polymer used is calcium alginate.

7. A process for the preparation of a microbial inoculum according to claim 5 or 6, characterised in that the microorganisms are symbiotic microorganisms of plants.

8. A process for the preparation of a microbial inoculum according to claim 7, characterised in that the microorganisms belong to the group of the Frankia or the Rhizobium.

9. A process for the preparation of a microbial inoculum according to any one of claims 6 to 8, characterised in that the polymer matrices obtained are in the form of balls or granules.

10. A process for the preparation of a microbial inoculum according to any one of claims 5 to 9, characterised in that the microorganisms from which the said inoculum is made are obtained by the two-phase culturing process according to any one of claims 1 to 4.

11. A microbial inoculum, characterised in that it is obtained by the process according to any one of claim 5 to 10.

12. Application of the microbial inoculum according to claim 11, characterised in that before use the polymer matrices are rehydrated with a buffer until a gel is obtained.

# FIG.1

1a

1b

# FIG.2

2a

2b